# EUROPEAN PATENT APPLICATION

(11) **EP 2 198 908 A1**
(43) Date of publication of application: **23.06.2010**
(21) Application number: 08022117.9
(22) Date of filing: 19.12.2008
(51) Int. Cl.: A61M 15/00

(54) **Inhaler**

(71) Applicant: BOEHRINGER INGELHEIM INTERNATIONAL GMBH, 55216 Ingelheim am Rhein (DE); Vectura Delivery Devices Limited, Chippenham, Wiltshire SN14 6FH (GB)
(72) Inventor: The designation of the inventor has not yet been filed
(74) Representative: Grey, Ian Michael

(57) **Abstract**

An inhaler (1) including a blister strip (2) is proposed. The unused blister strip is stored in a spiral (16). After use, the blister strip is conveyed into another spiral (17). Both spirals share a common space within the inhaler and may be separated by a loose guiding element (18). The spirals and the guiding element may comprise protrusions (19) to reduce sliding friction. Additionally or alternatively, a lubricant (21) may be used to reduce friction.

## Description

The present invention relates to an inhaler according to the preamble of claim 1, 3, 5 or 6.

The present invention relates to passive inhalers, i.e. inhalers where the patient or user breathes in to generate an air stream, which entrains the inhalation formulation and forms the desired aerosol.

The present invention relates to an inhaler for delivery of a powder-form inhalation formulation from a blister strip with a plurality of blister pockets (also called blisters) containing the inhalation formulation in doses.

GB 2 247 042 A discloses an inhaler with a rolled-up blister strip. The inhaler comprises a manually operated, pivotable actuator, which operates a conveyor for stepwise moving the blister strip. The actuator supports a piercer and an associated mouthpiece. By pivoting the actuator, the blister strip and be moved forward and blister pockets of the blister strip can be pierced one after the other. When a patient breathes in an air stream passes through the previously pierced blister pocket, with the result that the inhalation formulation in the blister pocket mixes with the air and is discharged to the patient. Depending on the length of the blister strip and on the transmission, operation of the actuator may require undersigned high forces.

Object of the present invention is to provide an inhaler wherein the operation is facilitated.

The above object is achieved by an inhaler according to claim 1, 3, 5 or 6. Preferred embodiments are subject of the subclaims.

According to a first aspect of the present invention, the inhaler comprises a guiding element preferably with a guiding surface for guiding preferably a contact side of a carrier, such as a blister strip. The surface comprises at least one protrusion protruding from the surface towards the contact side in order to reduce the areal contact and, thus, the friction between the guiding element and the carrier, i,e, the sliding friction can be reduced when the carrier is moved onward. This facilitates movement of the carrier and, thus, operation of the inhaler.

According to a second aspect of the present invention, the inhaler comprises a flexible guiding element for the unused carrier in a reservoir and preferably a flexible guiding element for the used carrier in a receiving part. This facilitates unwinding of the unused carrier and, in particular, winding-up of the used carrier and/or reduces or prevents the interference of the roll of unused carrier and the roll of used carrier in the inhaler. This facilitates movement of the carrier and, thus, operation of the inhaler.

According to a third aspect of the present invention, the inhaler comprises a loose guiding element between a reservoir for the unused carrier and a receiving part for the used carrier in order to reduce the friction between the carrier in the reservoir or a flexible spiral guiding the carrier in the reservoir on one hand and the carrier in the receiving part or a flexible spiral guiding the carrier in the receiving part on the other hand. This facilitates movement of the carrier and, thus, operation of the inhaler.

According to a fourth aspect of the present invention, an inhaler comprises a lubrication device and/or the carrier comprises a lubricant in order to reduce friction between the guiding element and the carrier. This facilitates movement of the carrier and, thus, operation of the inhaler.

Further aspects, features, properties and advantages of the present invention will be apparent from the claims and the subsequent description of a preferred embodiment, with reference to the drawings. There are shown in:
- Fig. 1: a schematic sectional view of an inhaler without mouthpiece cover;
- Fig. 2: a schematic sectional representation of the inhaler with closed mouthpiece cover;
- Fig. 3: a schematic sectional view of a guiding element of the inhaler;
- Fig. 4: a schematic sectional view of another guiding element of the inhaler; and
- Fig. 5: a schematic sectional view of a further guiding element of the inhaler.

In the Figures, same reference numbers are used for identical or similar parts, even if a repeated description is omitted. In particular identical or corresponding advantages and properties then also result or may be achieved.

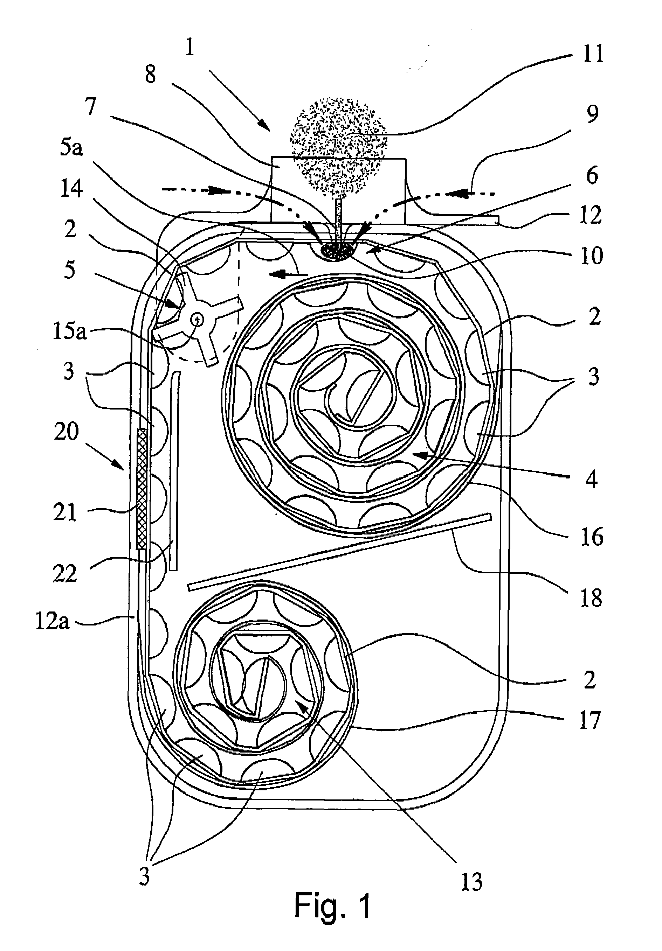

Fig. 1 shows in a very schematic sectional representation an inhaler 1.

The inhaler 1 serves to deliver a powdered inhalation formulation from a preferably band-shaped carrier, such as a blister strip 2. The blister strip 2 is finite, not forming an endless or closed loop. It has a large number of blister pockets 3 respectively containing directly a dose of the loose inhalation formulation, in particular powder 10, Thus, the formulation is pre-metered.

The inhaler 1 has a reservoir 4 for the still unused blister strip 2 with closed (sealed) receptacles, here blister pockets 3. The unused blister strip 2 is preferably rolled up or wound up or stored in the reservoir 4.

The inhaler 1 has preferably a conveyor 5 for stepwise onward movement of the blister strip 2 in direction of arrow 5a stepwise by one blister pocket 3, in order to feed the blister pockets 3 successively to an opening and/or removal position 6 where the respective blister pocket 3 is opened and can be emptied.

The blister pockets 3 can be opened respectively preferably by means of a removal device or piercing member 7, which punctures or cuts open a lid of the respectively aligned blister pocket 3 in position 6. The piercing member 7 is hollow and/or in fluid connection with an adjacent mouthpiece 8 of the inhaler 1.

During or for inhalation a patient or user, not represented, places the mouthpiece 8 in his mouth and breathes in. An air stream 9 of ambient air is sucked in and passes through the opened blister pocket 3 such that the loose powder 10 (forming the inhalation formulation and being schematically shown in Fig. 1 only in the actually opened blister pocket 3 below mouthpiece 8) is dispensed with the sucked-in ambient air as an aerosol cloud 11 via the mouthpiece 8. This situation is schematically represented in Fig. 1. Thus, the respectively opened blister pocket 3, into which the piercing member 7 partly extends, is emptied.

The inhaler 1 has a preferably manually actuateable, lever-like actuator 12 being pivotally mounted to a housing 12a of the inhaler 1. The piercing member 7 and the mouthpiece 8 are attached to and supported by the actuator 12.

The actuator 12 is operable (pivotable) to cause the piercing member 7 to puncture the lid of the respectively aligned blister pocket 3 in position 6 below the mouthpiece 8.

When the actuator 12 swivels from the position shown in Fig. 1 (here anticlockwise) to a partially opened position, the piercing member 7 is withdrawn from the last-pierced blister pocket 3.

Then, the blister strip 2 is moved forward by one blister pocket 3, so that the next blister pocket 3 is moved in position 6. This will be explained in more detail later.

When the actuator 12 swivels back into the position shown in Fig. 1, i.e, is manually moved back, the next aligned blister pocket 3 of the blister strip 2 is punctured by the piercing member 7 and thereby opened. Then, the next inhalation can take place, i.e. the inhaler 1 is activated.

The inhaler 1 has preferably a receiving space or part 13 to receive or store the used part of the blister strip 2. The receiving space or part 13 is formed such that the used part can be wound up. Fig. 1 shows a situation with essentially filled reservoir 4 and partly empty receiving space 13.

The conveyor 5 comprises preferably a conveying wheel 14, which can engage between the blister pockets 3 and thus convey the blister strip 2 in form-locking or form-fit manner. This allows very secure or precise moving or indexing of the blister strip 2 as desired and/or necessary.

The conveyor 5 or its conveying wheel 14 is preferably arranged preferably between the reservoir 4 and the receiving part 13, in particular between the removal position 6 and the receiving part 13, thus after the emptying of the blister pockets 3.

The pivot axis of the actuator or lever 12 is preferably coaxial with the rotation axis of the conveying wheel 14. In particular, the actuator or lever 12 may be supported by an axle of the conveying wheel 14.

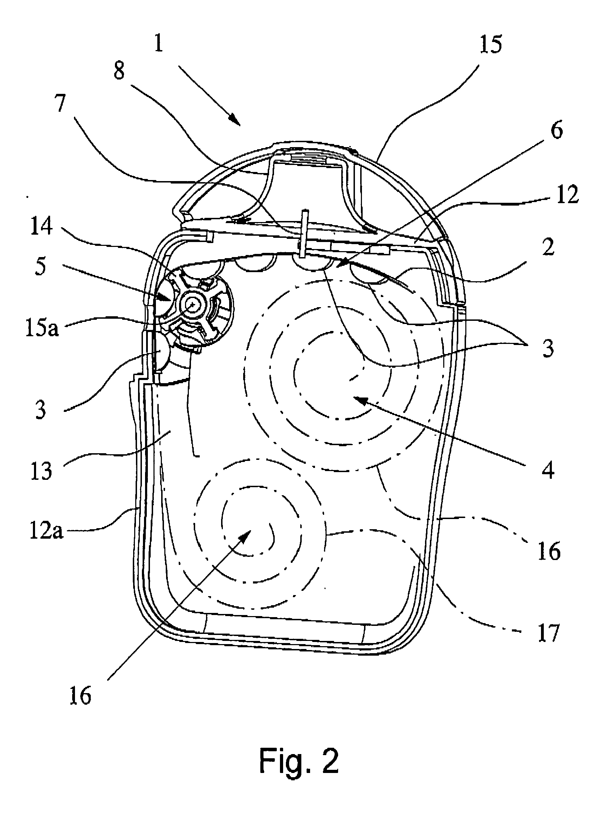

The inhaler 1 comprises a mouthpiece cover 15. The mouthpiece cover 15 is not shown in Fig. 1, which explains some aspects of the inhaler 1, but in Fig. 2, which shows a more realistic, but still very schematic sectional view of the inhaler 1. Fig. 2 shows the inhaler 1 with closed mouthpiece cover 15, wherein the blister strip 2 has been partly omitted for illustration purposes.

The mouthpiece cover 15 is pivotable around a cover axis 15a, which is indicated in Fig. 1 and 2 and extends perpendicular to the drawing plane in the present representation.

The pivot axis of the actuator 12 extends preferably coaxial to or with the cover axis 15a. The rotation axis of the conveying wheel 14 extends preferably coaxial to the cover axis 15a and to the pivot axis of the actuator 12.

The conveyor 5 or its conveying wheel 14 is preferably driven by the mouthpiece cover 15, namely by the pivotal movement of the mouthpiece cover 15. In particular, the blister strip 2 is moved forward, when the mouthpiece cover 15 is opened. Preferably, only part of the opening movement of the mouthpiece cover 15 actuates or operates the conveyor 5 or its conveying wheel 14 to move the blister strip 2 forward.

When the mouthpiece cover 15 is opened starting with the completely closed position shown in Fig. 2, in a first phase of the opening movement, for example up to a first angle of about 10, 20 or 30 degrees, the blister strip 2 is not moved due to a respective freewheel (not shown) between the mouthpiece cover 15 and the conveying wheel 14.

First of all, the actuator 12 has to be moved or opened in order to withdraw the piercing member 7 from the previously pierced and usually already emptied blister pocket 3. This opening movement of the actuator 12 can be performed manually. However, the actuator 12 preferably opens automatically when opening the mouthpiece cover 15. In particular, the mouthpiece cover 15 can be opened up to the first angle. When the mouthpiece cover 15 reaches this angle, e.g. about 20 degrees, the actuator 12 flips automatically open into its opened position, in particular due to a biasing or spring means (not shown) or the like. However, it also possible that the actuator 12 moves jointly with the mouthpiece cover 15 in the first phase of the opening movement (e.g. due to a ratchet mechanism, spring or the like) until the actuator 12 reaches its opened position or the first angle.

The opened position of the actuator 12 is preferably set such that the piercing member 7 is not exposed to the exterior and/or that the inhaler 1 is not completely opened in order to avoid or at least minimize any potential external influences.

In order to limit the open position of the actuator 12, the opening or pivot range of the actuator 12 is smaller than the one of the mouthpiece cover 15 and/or is restricted to preferably at most 20 degrees, in particular to about 10 degrees or less.

However, it is also possible that the actuator 12 is not limited in its opening position, but can open or pivot as far as the mouthpiece cover 15, in particular jointly with the mouthpiece cover 15.

During the further opening (second phase) of the mouthpiece cover 15, the conveyor 5 or its conveying wheel 14 is actuated to move or index the blister strip 2 by one blister pocket 3 onward to the next blister pocket 3 that shall be emptied. This blister movement happens preferably up to the complete opening of the mouthpiece cover 15.

When the mouthpiece cover 15 has been fully opened and the next blister pocket 3 has been moved in position 6, the actuator 12 can be pivoted back, i.e. closed, in order to pierce the already aligned, still closed blister pocket 3. Then, the inhaler 1 is ready for inhalation, i.e, activated as already described.

After inhalation, the inhaler 1 can be closed by pivoting back the mouthpiece cover 15 into its closed position.

Preferably, the inhaler 1 comprises a guiding element 16 for the unused carrier. The guiding element 16 is located in particular in the reservoir 4 or forms the reservoir 4, as shown in Fig. 1.

The guiding element 16 stores and/or guides the unused carrier, However, the guiding element 16 and/or an other guiding element 17 can be used to guide other parts of the carrier and/or can be located in other parts of the inhaler 1.

In the present embodiment, the inhaler comprises preferably an other guiding element 17. In particular, the other guiding element 17 is located in the receiving part 13 or forms the receiving part 13.

Additionally or alternatively, the inhaler 1 may comprise a further guiding element 18 as shown in Fig. 1, The further guiding element 18 is loosely received in the inhaler 1 or its housing 12a. The further guiding element 18 is located between the reservoir 4 and the receiving part 13 for reducing the friction between the carrier in the reservoir 4 or a flexible spiral (guiding element 16) guiding the carrier in the reservoir 4 on one hand and the carrier in the receiving part 13 or a flexible spiral (guiding element 17) guiding the carrier in the receiving part 13 on the other hand.

Preferably, the guiding elements 16, 17 and/or 18 are flexible and/or moveable or bendable when the carrier is moved or conveyed, in particular transversally to the carrier.

Preferably, the guiding elements 16, 17 respectively comprise a spiral portion or form at least essentially a spiral.

In particular, the guiding element 16 and/or 17 may encompass the carrier peripherally, preferably such that the roll of unused carrier in the reservoir 4 and the roll of used carrier in the receiving part 13 do not directly contact. This allows reduction of friction between these rolls. This may be achieved alternatively or additionally by the guiding element 18.

The other guiding elements 16, 17 and/or 18 are preferably also strip- or band-like.

Preferably, the guiding elements 16, 17 and/or 18 are made of steel, in particular spring steel. However, the guiding elements 16, 17 and/or 18 can also be made of any other suitable material, in particular plastics.

Preferably, the guiding element 16, 17 and/or 18 are made from sheet material, in particular metal sheet.

The spiral / guiding element 16 of the reservoir 4 may be accommodated in the housing 12a loosely. Then, the outer end of the guiding element 16 preferably engages the housing 12a, a recess or the like in order to counterbear the guiding element 16 when the carrier is drawn out of the spiral / guiding element 16 during the onward movement 5a of the carrier.

The spiral / guiding element 17 may be filed at one part or end, preferably an outer end, to the housing 12a or any other stationary component of the inhaler 1. This applies for the other guiding elements 16 and 18 as well.

Preferably, the reservoir 4 and the receiving part 13 are very close and, in particular, share a common space within the inhaler 1 / housing 12a in order to allow a very compact design of the inhaler 1. In particular, the spiral of the reservoir 4 is large in diameter before first use of the inhaler 1 (in this state, most of the carrier is located in the reservoir 4). In this state, the receiving part 13 requires nearly no space because the carrier has not yet been moved into the receiving part 13. Therefore, the spiral of the receiving part 13 can be minimal in diameter in this state.

During use of the inhaler 1, the size of the roll of the unused carrier and/or the spiral decreases in the reservoir 4 and increases in the receiving part 13. Thus, the receiving part 13 is shifted during the further use of the inhaler 1 towards the reservoir 4. Correspondingly, the loose guiding element 18 is moved or shifted during the use of the inhaler 1, more precisely with the onward movement of the carrier from the reservoir 4 into the receiving part 13.

In order to (further) reduce the friction, any one of the guiding elements 16, 17, 18 may comprise at least one protrusion 19 protruding towards the carrier or spiral, in particular protruding from an at least essentially flat surface or side of the respective guiding element 16, 17, 18 towards a preferably essentially flat contact side of the carrier (in particular, the side of the carrier where the blister pockets 3 are sealed by a foil or the like). Thus, it is possible to reduce the areal contact between the guiding element 16, 17, 18 on one hand and the associated carrier or spiral or one of the other guiding elements 16, 17, 18 on the other hand. With other words, the protrusion(s) 19 may reduce friction between guiding element and carrier or between two guiding elements.

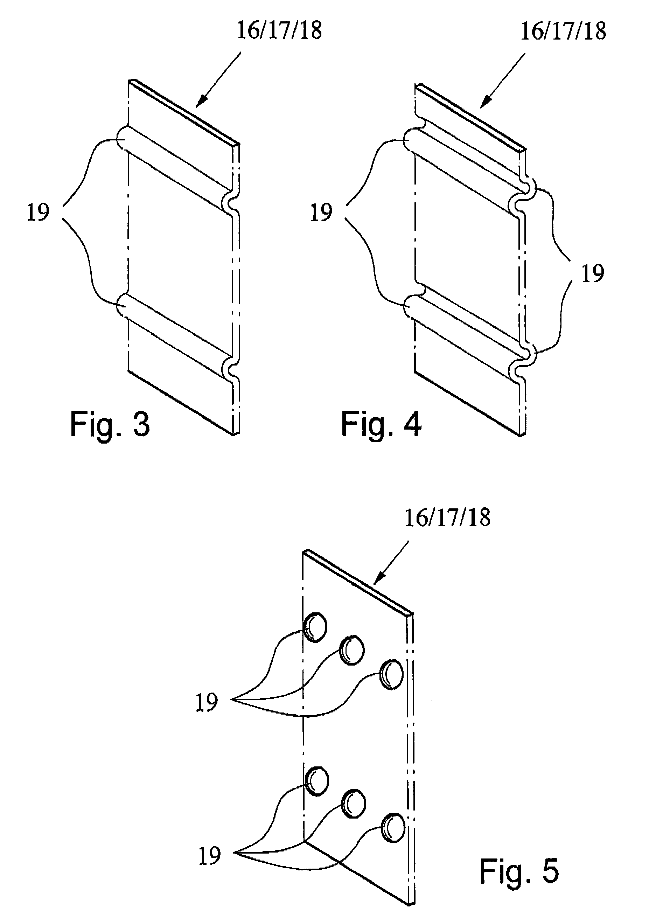

Fig. 3 shows in a partial sectional view part or one guiding element 16, 17 or 18 with multiple protrusions 19, here two protrusions 19 on one side or surface. However, only one protrusion 19 can be provided on one side if desired.

The at least one protrusion 19 is preferably rib-like and/or rail-like and/or extends in lengthwise direction of the guiding element or carrier.

If two protrusions 19 are provided and extend in lengthwise direction, the distance between the protrusions 19 and the location of the protrusions 19 is choosen such that the associated carrier is securely guided on or between the protrusions 19.

The protrusions 19 guide or contact preferably the flat contact side or backside of the carrier. However, the protrusions 19 can also guide or contact or support the carrier on its other side, in particular such that the receptacles /blister pockets 3 engage inbetween the protrusions 19.

The guiding element 16, 17 or 18 may comprise protrusions 19 on opposite sides as shown in Fig. 4. This may reduce the friction on both sides to the carrier, in particular if the carrier is guided in spiral form and, thus, may contact both sides of the respective guiding element 16, 17 or 18. However, the two - sided location of protrusions 19 may be used for other purposes as well, e.g. to reduce the friction on one side of the guiding element 16, 17 or 18 with a carrier and on the other side of the guiding 16, 17 or 18 with one of the other guiding elements 16, 17 or 18 or the like.

The guiding elements 16, 17 or 18 may comprise multiple protrusions 19 preferably evenly distributed in lengthwise direction and/or protrusions 19 with essentially hemispherical form as shown in Fig. 5 or with any other form.

The protrusions 19 are formed preferably by corrugations of the respective guiding element 16, 17 or 18 and/or by deep-drawing or the like. However, the protrusions 19 could also be formed by folding, e.g. of edges of the respective guiding element 16, 17 or 18 or the like.

Additionally or alternatively to the above possibilities to reduce friction, the inhaler 1 may comprise a lubrication device 20 and/or the carrier may comprise or be provided with a lubricant 21 in order to reduce friction between at least one of the guiding elements 16, 17 or 18 and the carrier, as schematically shown in Fig 1. The lubrication device 20 may be formed by a suitable material, such as felt or the like, which is soaked with the lubricant 21 and is in contact with the carrier or any other component in order to transfer the lubricant 21 to the carrier or the other component.

Preferably, the lubricant 21 is applied to the carrier after the opening and/or removal position 6 and/or on the side opposite of the removal side of the carrier.

As mentioned, the lubricant 21 may be applied to the carrier, in particular within the inhaler 1, preferably by means of the lubrication device 20. However, it is also possible that the lubricant is applied to the carrier before the carrier is inserted into the inhaler 1. In particular, the lubricant 21 may form a coating of the carrier or any guiding element or any other component of the inhaler 1. The lubricant 21 may be applied more or less to one side of the carrier and/or only in one part of the carrier, e.g. the end region of the carrier which is inserted into the receiving part 13 at first.

Preferably, the lubricant 21 is viscous and/or adheres to the carrier.

The guiding elements 16, 17 and 18 described above are preferably - at least partially - flexible, moveable and/or elastically deformable, in particular transversally to a flat side and/or to the associated carrier. However, the inhaler 1 can additionally or alternatively also comprise a rigid and/or stationary guide 22 for guiding the carrier and/or partitioning the reservoir 4 and the receiving part 13 against each other, as schematically shown in Fig. 1. This guide 22 can also be provided with at least one protrusion 19 as described above in order to reduce the (sliding) friction with the carrier and/or one other guiding element 16, 17 or 18, in particular a spiral formed thereof.

Preferably, the inhaler 1 is portable, works only mechanically and/or is handheld.

Preferably, the terms "blister strip" and "blister pockets" have to be understood in a very broad sense to cover also other kinds of storage means with receptacles or even bulk storages for the formulation.

### List of reference numbers

- 1: inhaler
- 2: blister strip
- 3: blister pocket
- 4: reservoir
- 5: conveyor
- 5a: onward movement
- 6: opening and/or removal position
- 7: piercing member
- 8: mouthpiece
- 9: air stream
- 10: powder
- 11: aerosol cloud
- 12: actuator
- 12a: housing
- 13: receiving part
- 14: conveying wheel
- 15: mouthpiece cover
- 15a: cover axis
- 16: guiding element
- 17: guiding element
- 18: guiding element
- 19: protrusion
- 20: lubrication device
- 21: lubricant
- 22: guide

## Claims

1. Inhaler (1) for delivering a preferably powdered formulation from a band-shaped carrier, in particular a blister strip (2), with an essential flat contact side and a plurality of receptacles, such as blister pockets (3), containing the formulation in doses, comprising:
a conveyor (5) for moving the carrier onwards; and
a guiding element (16, 17, 18) the carrier when the carrier moves;
**characterized in**
**that** the guiding element (16, 17, 18) comprises at least one protrusion (19) protruding from the towards the carrier in order to reduce the areal contact and, thus, the friction between the guiding element (16, 17, 18) and the carrier.

2. Inhaler according to claim 1, **characterized in that** the inhaler (1) comprises a reservoir (4) for storing the used carrier, the guiding element (16, 17, 18) being arranged in or forming the reservoir (4).

3. Inhaler (1) for delivering a preferably powdered formulation from a band-shaped carrier, in particular a blister strip (2), with a plurality of receptacles, such as blister pockets (3), containing the formulation in doses, comprising:
a reservoir (4) for storing the unused carrier; and
a receiving part (13) for the used carrier;
**characterized in**
**that** the inhaler (1) comprises a flexible guiding element (16, 17, 18) for the unused carrier in the reservoir (4) and preferably a flexible guiding element (16, 17, 18) for the used carrier in the receiving part (13).

4. Inhaler according to claim 3, **characterized in that** the guiding element(s) (16, 17) comprise(s) at least one protrusion (19) protruding towards the carrier in order to reduce the areal contact and, thus, the friction between the guiding element (16, 17) and the carrier.

5. Inhaler (1) for delivering a preferably powdered formulation from a band-shaped carrier, in particular a blister strip (2), with a plurality of receptacles, such as blister pockets (3), containing the formulation in doses, preferably according to one of the previous claims, comprising:
a reservoir (4) for storing the unused carrier;
a receiving part (13) for receiving used or opened carrier; and
a conveyor (5) for moving the carrier from the reservoir (4) into the receiving part (13),
**characterized in**
**that** the inhaler (1) comprises a loose guiding element (18) between the reservoir (4) and the receiving part (13) for reducing the friction between the carrier in the reservoir (4) or a flexible spiral guiding the carrier in the reservoir (4) on one hand and the carrier in the receiving part (13) or a flexible spiral guiding the carrier in the receiving part (13) on the other hand.

6. Inhaler (1) for delivering a preferably powdered formulation from a band-shaped carrier, in particular a blister strip (2), with a plurality of receptacles, such as blister pockets (3), containing the formulation in doses, preferably according to one of the previous claims, comprising:
a conveyor (5) for moving the carrier onwards; and
a guiding element (16, 17, 18) for guiding the carrier when the carrier moves;
**characterized in**
**that** the inhaler (1) comprises a lubrication device (20) and/or a the carrier comprises a lubricant (21) in order reduce friction between the guiding element (16, 17, 18) and the carrier.

7. Inhaler according to claim 5 or 6, **characterized in that** the guiding element (16, 17, 18) comprises at least one protrusion (19) protruding towards the carrier or spiral in order to reduce the areal contact and, thus, the friction between the guiding element and the carrier or spiral.

8. Inhaler according to claim 1, 2, 4 or 7, **characterized in that** the protrusion (19) extends in or along a lengthwise direction of the guiding element (16, 17, 18) or carrier.

9. Inhaler according to claim 1, 2, 4, 7 or 8, **characterized in that** the protrusion (19) is rail- or rib-like.

10. Inhaler according to claim 1, 2, 4, 7, 8 or 9, **characterized in that** the protrusion (19) is formed by a corrugation of the guiding element (16, 17, 18).

11. Inhaler according to one of the previous claims, **characterized in that** the guiding element (16, 17, 18) comprises one or two protrusions (19) extending parallel in lengthwise direction (5a), and/or that the guiding element (16, 17, 18) comprises multiple protrusions (19) distributed in a lengthwise direction of the guiding element (16, 17, 18).

12. Inhaler according to one of the previous claims, **characterized in that** the guiding element (16, 17, 18) comprises protrusions (19) on opposite sides of the guiding element (16, 17, 18).

13. Inhaler according to one of the previous claims, **characterized in that** the guiding element (16, 17, 18) is made of steel, in particular spring steel, and/or that the guiding element (16, 17, 18) is made from sheet material, in particular metal sheet, and/or that the guiding element (16, 17, 18) is flexible or elastic or moveable or bendable transversally to the carrier.

14. Inhaler according to one of the previous claims, **characterized in that** the guiding element (16, 17, 18) encompasses the carrier peripherally.

15. Inhaler according to one of the previous claims, **characterized in that** the guiding element (16, 17, 18) comprises a spiral portion or at least essentially the form of a spiral.
